# EUROPEAN PATENT APPLICATION

(11) **EP 2 329 836 A1**
(43) Date of publication of application: **08.06.2011**
(21) Application number: 09425497.6
(22) Date of filing: 03.12.2009
(51) Int. Cl.: A61K 36/27, A61P 3/04, A61P 3/10, A61P 31/10, A61P 1/04, A61K 8/00, C12N 5/04, A01H 4/00

(54) **Extracts obtained from hoodia gordonii cells lines, their preparation and use**

(71) Applicant: I.R.B. Istituto Di Ricerche Biotecnologiche S.r.l., 36077 Altavilla Vicentina (VI) (IT)
(72) Inventor: Dal Toso, Roberto, 36077 Altavilla Vicentina (VI) (IT); Pressi, Giovanna, 36077 Altavilla Vicentina (VI) (IT); Manfredini, Stefano, 36077 Altavilla Vicentina (VI) (IT)
(74) Representative: Long, Giorgio

(57) **Abstract**

The present invention refers to the preparation and use of the extracts for nutritional, pharmacological and cosmetic purposes. Said extracts are obtained from cell cultures of *Hoodia gordonii* IRB HGORD 42 having accession number DSMZ: DMS 17433. Furthermore, the present invention concerns the preparation and use of said extracts, for the production of drugs or nutritional or cosmetic substances, such extracts possessing appetite suppressant properties.

## Description

### FIELD OF THE INVENTION

The present invention refers to the preparation and use of the extracts for nutritional, pharmacological and cosmetic purposes. Said extracts are obtained from "in vitro" cell cultures of *Hoodia gordonii* (*Asclepiadaceae* family). Therefore, the present invention concerns the preparation and use of said extracts, for the production of drugs or nutritional or cosmetic substances, such extracts possessing particularly appetite suppressant properties.

### STATE OF THE ART

Data reported in patents (US2003/0152648A1; US6,376,657B1; US2002/0146468A1; US6,488,967B1) and in literature (MacLean D.B., Lu-Guang Luo. Increased ATP content/production in the hypothalamus may be a signal for energy-sensing of satiety: studies of the anorectic mechanism of a plant steroidal glycoside. Brain Research, 2004, 1020: 1-11) show that extracts obtained from plants belonging to the genus *Hoodia, Trichocaulon, Orbea* and *Stapelia* (Asclepiadaceae family) comprise substances having appetite suppressant activity. Such substances belong to the steroidal glycosides group. Particularly, US patent 6,376,657 B1 discloses a purification process to isolate a molecule belonging to the class of pregnanglycosides, called P57, having a molecular weight 878 and having appetite suppressant activity.

In US patent 2002/0146468A1 it has been shown that extracts obtained from plants of the genus Hoodia and Trichocaulon have anti-diabetic activity in type II diabetes mellitus. Also, in the US patent 2002/0146468A1 it has been reported that extracts from plant of the genus Trichocaulon and of the genus Hoodia are effective in the treatment of gastric hypersecretion pathologies (gastric and duodenal ulceration, reflux esophagitis and hyatus hernia).

*Hoodia gordonii* plants and other genus belonging to the class of Asclepiadaceae grow only in the dry regions and especially in the Kalahari desert situated in the South-Africa region. These plants reach the ripeness within a long time span between 5 and 7 years of the life. Furthermore, such plants have a limited supply due to the growth difficulties in other geographical areas. Compounds having appetite suppressant activity present in *Hoodia gordonii* are contained in low quantities in other plants of the same family present also in the deserts of North America and Mexico. For these reasons, the availability of the plant is limited for the preparation of the extracts and the price of the extracts is thus very high for commercial purposes.

The technical problem of the present invention is to set up a process for manufacturing extracts from *Hoodia gordonii,* which allows to obtain industrial amounts of active substances having pharmacological, cosmetical or nutritional activity.

Such a problem is solved by a method as disclosed in the main claim of the present application.

### SUMMARY OF THE INVENTION

A first object of the invention is a process for the production of extracts having appetite suppressing activity from *Hoodia gordonii,* which allows to obtain industrial amounts of active extracts.

A second object of the invention relates to active extracts of said plant cells for use in the preparation of drugs, nutritional or cosmetic substances.

A third object of the invention is the availability of a selected and stable *Hoodia gordonii* cell line.

Further objects, characteristics and the advantages of the present invention will be clearly understood from the following detailed description of the invention.

### DETAILED DESCRIPTION OF THE INVENTION

A number of experiments to obtain large amounts of active extracts of *Hoodia gordonii* have been performed to solve the technical problem above described.

Generally, the process comprises the following stages in sequence:
1) collecting of the plant biomass obtained from Hoodia gordonii cell culture on solid medium or liquid medium;
2) selection of the clone deriving from the cell culture with highest biological activity;
3) homogenisation of the cells;
4) separation of the cell residues from the aqueous phase (supernatant);
5) lyophilization of the supernatant.

Preferably, the collection stage of the biomass is made after the fermentation of the cells obtained from *Hoodia gordonii* plant comprising a first selection stage of cell cultures on the basis of the proliferation rate and of the biological activities.

Briefly, this process envisages the collection of plant tissue, particularly from the stems, the cleaning thereof, for example under running water, cutting into fragments of 2-5 cm and sterilisation on plates, for example by sequential treatment with 70% ethanol for approx. 15 minutes, 2% sodium hypochlorite for approx. 5 minutes and finally 0.05% HgCl₂ for approx. 1 minute. Between treatments, the plant fragments are washed, typically three or more times with sterile distilled water.

Each framment of said tissue, chopped-up even further (explants), is placed on a Petri dish containing nutrient medium, solidified by the addition of Agar and supplemented with growth hormones without the addition of any antibiotics. The number of explants performed influences the outcome of the subsequent stages. Generally from 2000 to 5000 uncontaminated explants are sufficient to proceed to the subsequent selection stage.

Following a suitable period of time, undifferentiated callus tissue forms, which is then multiplied following transfer onto a greater surface area with fresh medium.

Preferably, the plant cell line derived from the undifferentiated callus tissue is preferably stabilised by means of a certain number of transfers (sub-cultures) onto fresh culture medium. Particularly, it has been observed that in order to obtain a stable cell line, it is important to perform at least ten sub-cultures. Such medium is solid in nature, and may be advantageously constituted by 0.8-1% agar in a standard culture medium, to which has been added plant peptone, allowing a balanced supply of aminoacids and guaranteeing the maintenance of good cell wall integrity. Preferably, plant peptone will be added in quantities ranging between 500 and 4000 mg/l of culture medium.

A "stable cell line" is defined as a culture having the following characteristics:
- high and constant proliferation rate over time;
- preservation of the same phenotypic characteristics throughout various subcultures (cell colour, aggregate friability, size etc.);
- reproducible biological activity concerning appetite suppressant properties and anti-fungal activity throughout various subcultures;
- constant primary metabolite content (protein, lipids and polysaccharides) per unit mass.

Subsequently, at the stabilisation stage, the cell line is subjected to "clonal selection". Such selection consists of growing the stabilised cells for an appropriate amount of time (typically, 10-15 days of culture). Subsequently, individual cell aggregates are taken from the solid culture medium and each of said cell aggregates is inoculated into liquid culture medium described above.

Following fermentation for such time necessary to obtain adequate multiplication of the cell aggregate (hereinafter referred to as "clone"), a time generally comprised of between 10 and 15 days, the biological activity of interest is determined for each clone.

Such operations may be repeated until a plant cell line clone is selected, in which the biological activity of interest is the greatest.

It should be remembered that alternating periods of culture on solid and liquid media is essential for the clonal selection process of the present invention. Hence, it is essential that the clonal selection process described above does not conclude with the identification of the most active clone, but is constantly repeated so as to keep the selected clone phenotypically homogeneous.

The selected plant cell line is then multiplied in order to obtain a sufficient quantity of biomass to carry out the production fermentation stage. Said quantity will depend on the specific production requirements, the plant cell line typology used and the type of biological activity it is desired to maximize.

The biomass thus obtained may be passed directly into the final fermenter, or can be subjected to one or more further growth stages in liquid medium, working with intermediate volumes.

Preferably, the process of clone selection includes the stages of:
a)cultivating a predetermined plant cell line, stabilised for a sufficient period of time to allow the multiplication of said cell line to give substantially distinct cell clusters, on solid medium;
b)removing said substantially distinct cell clusters from said solid medium and placing each of them in a separate liquid culture medium;
c)cultivating each of the said substantially distinct cell clusters in said liquid culture medium for a sufficient period of time to allow the multiplication of said cell clusters, and the determination of the biological activity;
d)performing a qualitative and quantitative determination of biological activity produced by each of said cell clusters in said liquid culture medium;
e)selecting the cell cluster capable of performing the greatest biological activity;
f) repeating the process cycle according to stages a), b), c), d) and e) on said cell cluster, selected according to stage e), a sufficient number of times until the biological activity of interest produced by the selected cell cluster, and by the cell cluster deriving from further selection cycles, is essentially constant.

In addition, the subsequent fermentation may preferably consist of the following stages:
A) the inoculation of said plant clone into liquid medium and the multiplication thereof for a sufficient period of time to obtain an increase in cellular mass of at least 300% of the weight thereof;
B) optionally, transfer of the suspension obtained from stage A) into fresh liquid culture medium and the multiplication thereof for a sufficient period of time to obtain an increase in cellular mass of at least 300% of the weight thereof;
C) optionally, the repetition of stage B) at least one additional time;
D) the transfer of the suspension obtained in stages A), B) or C) into fresh liquid culture medium in a fermenter to give a biomass, and conducting the fermentation under such conditions and for a sufficient period of time so as to obtain within said biomass, the production of the biological activity of interest.

In accordance with one preferred embodiment, the fermentation will normally be performed at a temperature of between 15°C and 35°C, typically around 25°C and for a period of time normally between 7 and 40 days, preferably between 14 and 21 days. It is essential that the biomass be adequately aerated and that at the same time be kept stirred by means of stirring external to the fermenter. Indeed, it has been observed that plant biomass is comprised of cells having cell-walls that are poorly resistant to rupture. A stirrer submerged into the biomass acts mechanically on the cells and can easily cause the lysis thereof. However, it is necessary that the stirring, although delicate, be efficient, above all in the final fermentation stages when the biomass greatly increases in density. For the purposes of the present invention, particularly appropriate methods of stirring are orbital means of stirring. Such means of stirring preferably operate at 40-200 rpm, more preferably at around 120 rpm.

It is appropriate that the volume of the container (fermenter) in which the fermentation occurs be considerably greater than the volume of the biomass. Typically, the volume of the reactor will be from 50% to 200% greater than the biomass volume.

As already mentioned, efficient fermentation requires adequate oxygenation. Oxygenation is normally performed by using sterile air with a flow rate of 0.5-4 1/minute, more preferably 2-2.5 1/minute, for a volume of 10 litres of biomass. Alternatively, gas mixtures containing from 10% to 100% v/v of oxygen may be used.

As mentioned previously in relation to stirring, even oxygenation by means of over violent bubbling can cause rupturing of the cell walls. Hence it is necessary to ensure that oxygenation is performed delicately, for example by bubbling through appropriate diffusers. It will be preferable to use means of air or oxygen diffusion with nozzle delivery flow speeds comprised of between 10 m/min and 600 m/min, more preferably between 50 m/min and 350 m/min.

In addition, the shape of the fermentation chamber has significant importance. Indeed, it is recommended that it has a smooth and uniform surface, i.e. that there are no edges, corners or other parts which can cause the rupture of the cell walls of the biomass.

According to one particular embodiment of the present invention, additives increasing oxygen solubility in water will be added to the biomass. Such additives will preferably be selected from those substances defined as "artificial blood", for example the perfluorinated hydrocarbons (PFC).

Particularly, as a non-limiting example of the present invention, stabilized cell lines derived from *Hoodia gordonii* have been selected for their high rate of proliferation and for their anti-fungal and appetite suppressant properties.

By way of non-limiting example, the details of a *Hoodia gordonii* cell line, the extraction conditions and the extracts called "active extracts" thereof, will now be described.

### Morphological characteristics of the cell line

The *Hoodia gordonii* cell line denominated IRB HGORD42 DSMZ (Deutshe Sammlung von Mikroorganismen und Zellkulturen, Braunschweig) accession N°: DSM 17443 (accession date: 18-05 2005), stabilized and selected in accordance the above procedure is yellowish with brownish area and mucid (sticky) appearance.

### Extraction process

The active extracts obtained from Hoodia gordoni cell line have been prepared with the following procedure.

As mentioned previously, the procedure comprises the following stages in sequence:
1) harvesting of the plant biomass obtained from Hoodia gordonii cell culture on solid medium or liquid medium;
2) selection of the clone deriving from the cell line with greatest biological activity;
3) homogenisation of the cells;
4) separation of the cell residues from the aqueous phase (supernatant);
5) lyophilization of the supernatant.

### A. Preparation of an active extract (CELL S) from plant cell lines on solid medium

In particular, cell cultures of the stable and selected IRB HGORD42 cell line are harvested with ages comprised of between 7 and 21 days. The harvested cells are homogenised for a time comprised of between 5 and 30 minutes. the cell residues are separated from the aqueous phase by centrifugation with a number of RPM comprised between 1000 and 10000 for a period of time varying between 2 and 30 minutes and at a temperature comprised of between 10°C and 40°C. The aqueous phase is lyophilised. The lyophilised aqueous phase is the active extract called "CELL S".

### B. Preparation of an active extract (CELL L) from plant cell line on liquid medium

In accordance with a first embodiment, cultures in suspension of the IRB HGORD42 cell line are harvested with ages comprised of between 7 and 21 days. The cells are preliminary separated from liquid medium by means of gravity filtration, pressure filtration or centrifugation through a porous mesh, for example nylon, steel or cotton mesh etc., preferably having a porosity comprised of between 50 µm and 150 µm. The subsequent steps of the process are similar to the process described previously.

The lyophilised aqueous phase is the active extract called "CELL L".

### C. Preparation of an active extract (CELL LMW) containing low molecular weight compounds (<10KDa)

According to a further embodiment of the invention, cultures of the cell line IRB HGORD42 in suspension, harvested with ages comprised of between 7 and 21 days and extracted in accordance with the general process described in b),in addition, after the stage 2) of separation, the suspensions are filtrated by selective tangential filtration of the supernatant at a temperature comprised of between 10°C and 40°C and at a pressure of between 0,2 and 3 bar. Such filtration stage allows the separation of the compounds with a molecular weight lesser than 10 KDa from the compounds with a molecular weight higher than 10 KDa. The filtrated containing compounds with low molecular weight (<10KDa) is lyophilised as described previously. The lyophilisate obtained is the active extract called "CELL LMW". Preferably, the tangential filtration involves the use of filters with a cut-off comprised of between 3 and 30 KDa.

### D. Preparation of a butanol soluble active extract (CELL B)

In accordance with a further embodiment of the invention, cultures of the cell line IRB HGORD42 in supension, are harvested with ages comprised of between 7 and 21 days and extracted as described in the procedure B). After the stage 4) the lyophilisate obtained (CELL L) is extracted with methanol. To the lyophilisate is added methanol in weight/volume ratios comprised of between 1:1 and 1:50 for a period of time comprised of between 5 and 180 minutes. The methanol extract is dried. Subsequently, the dry methanol extract is further extracted with n-butanol. n-butanol is added to the dry methanol extract in weight/volume ratios comprised of between 1:5 and 1:100. The butanol extract is dried. Such dry extract is the active extract called CELL B.

### E. Preparation of a cell active extract (CELL T)

A further embodiment of the invention attainment is a procedure comprising the stages of:
- harvesting of the plant biomass obtained from a cell culture of plants of the Asclepiadaceae family;
- selection of the clone deriving from the cell line with highest biological activity;
- lyophilisation of the cells coming from said harvesting optionally together with culture medium.

Such an embodiment allows the recovery of the cell culture without any treatment unless the final preservation treatment through lyophilizing or drying process. In fact, it has been observed that, as reported later with the reference to the examples, also the cell preparation derived from culture is endowed with nutritional, pharmacological and cosmetic properties.

A similar active extract, from the point of view of the content, may also be obtained in accordance with the procedure A) where the separation stage is absent. In this case, the active extract comprises also the cell residues.

Further object of the invention is an active extract obtainable in accordance with any one of the procedures described previously. In particular, said active extract is obtainable from cell line of Hoodia gordonii called IRB HGORD42 having accession number DSM 17443 and accession date: 18-05 2005.

Further object of the invention is the cell line of *Hoodia gordonii* IRB HGORD42 DSMZ (Deutshe Sammlung von Mikroorganismen und Zellkulturen, Braunschweig) accession N°. DSM 17443 (accession date: 18-05 2005).

By way of non-limiting example, some embodiments of the extraction method of active extracts from stable and selected IRB HGORD42 cell line are reported.

### EXAMPLE 1 - PREPARATION OF THE "CELL S" ACTIVE EXTRACT

Selected and stable cells of the *Hoodia gordonii* IRB HGORD42 cell line, grown on solid medium (Gamborg B5 in 1% agar supplemented with 30g/L sucrose, 2g/L plant peptone, 1mg/L Picloram and 0,2 mg/L Benzylaminopurine at final pH 6,5) at a temperature of 25°C and in the dark for a period of 14 days, are harvested and homogenised using an ultraturrax for a time of 10 minutes. The suspension is then centrifuged to 4000 RPM for 15 minutes in order to separate the cell residues from the aqueous phase. The clear supernatant is then lyophilised. 5,22 g of lyophilisate are obtained from 500 g of cells collected from solid medium. The lyophilisate is the "CELL S" active extract.

### EXAMPLE 2 - PREPARATION OF THE "CELL L" ACTIVE EXTRACT

Selected and stable cells of the *Hoodia gordonii* IRB HGORD42 cell line, grown on solid medium (Gamborg B5 in 1% Agar, supplemented with 30g/L sucrose, 2g/L plant peptone, 1mg/L Picloram and 0,2 mg/L Benzylaminopurine at pH 6,5) are inoculated into 5 X 3L flasks, each containing 1L of liquid medium (Gamborg B5 supplemented with 30g/L sucrose, 2g/L plant peptone, 1mg/L Picloram and 0,2 mg/L Benzylaminopurine at pH 6,5). The cells are inoculated into liquid medium in a quantity of 5% (weight/volume). The suspensions are incubated on a rotary shaker (120 RPM) at a temperature of 25°C in the dark. After a period 14 days the plant biomass (5 L of cell suspension) is harvested and filtrated on a nylon porous mesh having a porosity of 50 µm. The collected cells are then homogenised using an ultraturrax for a time of 10 minutes and subsequently centrifuged to 4000 RPM for 15 minutes at a temperature of 25°C in order to remove the solid residue. The clear supernatant is then lyophilised. 56,85 g of lyophilisate are obtained from 5 litres of cellular suspension. The lyophilisate is the active extract called CELL L". Non-homogenised cells and only dried/lyophilised (CELL T) are used for comparison.

### EXAMPLE 3 - PREPARATION OF THE "CELL LMW" ACTIVE EXTRACT

The plant biomass is obtained as described in the example 2.

The plant biomass is extracted as described in the example 2 with the exception of the last stage. The clear supernatant, obtained after centrifugation, is ultrafiltrated through dialysis, working at a temperature of 25°C and at a pressure of 1 bar and using a membrane having a CUT OFF of 10 KDa. The permeate, containing compounds with molecular weight lesser than 10 KDa, is lyophilised.

40,49 g of lyophilisate, containing compounds with molecular weight lesser than 10 KDa, are obtained from 5 litres of cellular suspension. The lyophilisate is the active extract called "CELL LMW".

### EXAMPLE 4 - PREPARATION OF THE "CELL B" ACTIVE EXTRACT

50 g of the "CELL L" active extract, obtained as described in example 2, are treated with 200-1000 ml of methanol, typically 500 ml, in reflux conditions for a time of 20-60 minutes, preferably 30 minutes. The supernatant is quantitatively transferred in a flask and said operation is repeated from 1 to 5 times, preferably 3 times. The methanol phases are collected and dried. The final product is a brown, amorphous and gummy solid of 13,2 g. Subsequently, to the raw solid is added 100-500 ml of n-butanol, preferably 300 ml, and 50-250 ml of water, typically 150 ml. With the aim of making easier the dissolution and the ripartition of the solid between n-butanol and water, the solution is sonicated for 3-10 minutes, preferably 5. The two phases are transferred in a separation funnel and then were divided. The aqueous phase is extracted for 1 to 5 times, preferably 3, with 50-250 ml of n-butanol, typically 50 ml. The organic phase, after anhydrification with anhydrous sodium sulfate and filtration, is dried. The dried organic phase (3,9 g) is the "CELL B" active extract.

Active extracts, prepared as described in examples 1, 2, 3 and 4 have been used to perform the biological assays. Several examples, which are given by way of non-limiting illustration, of the appetite suppressing and anti-fungal activity are set forth herein below.

### EXAMPLE 5 - ASSAY OF APPETITE SUPPRESSING ACTIVITY OF CELL S AND CELL L ACTIVE EXTRACTS

To the purpose of assessing and of comparing the appetite suppressing activity of *Hoodia gordonii* plant extracts and *Hoodia gordonii* cell extracts, an assay has been performed, generally called "daily food intake". The extracts have been orally administered to eight groups of nine animals (mouse) each. The vehicle has been used as negative control. Extracts obtained from the tissue of the plant which has been used to generate the cell line and commercial extracts from *Hoodia gordonii* (in US patent 6,376,657 B1 it is reported an appetite suppressant activity of extracts from plants belonging to the species *Hoodia* gordonii) have been used as positive controls. Extracts have been orally administered in a 0,2 ml volume for each animal through an oral gavage 2 hr before the beginning of the dark cycle.

The following extracts have been administered:
1) C1 extract: plant commercial extract administered at a dosage of 600 mg/kg;
2) C2 extract: plant commercial extract administered at the dosage of 3000 mg/kg;
3) PL extract: plant extract administered at the dosage of 600 mg/kg;
4) CELL S1 extract: extract obtained from plant cell cultures on solid medium administered at the dosage of 600 mg/kg;
5) CELL S2 extract: extract obtained from plant cell cultures on solid medium administered at the dosage of 3000 mg/kg;
6) CELL L extract: extract obtained from plant cell cultures in liquid suspension administered at the dosage of 3000 mg/kg.
7) CELL T extract: total dried/lyophilisate obtained from plant cell cultures in suspension at the dosage of 3000 mg/Kg

This assay has been conducted with 72 female BALB/C mice 6 weeks aged (Charles River Laboratories, Indipendenza 11 street, Calco (LC), Italy). After the quarantine period, the animals have been separated in 8 treatment groups of 9 mice and in a control group of 9 mice. Mice have been housed individually in standard cages. The cages have been placed into a sterile insulator and in a controlled environment. Animal care was in compliance with standard procedures of good stalling practice. It was decided to host a single animal in a cage in order to adequately assess the actual daily consumption of food. At each animal has been administered the examination sample by oral gavage. Before of the administration of the extracts a quantity of fixed food, equal to 100 g, it has been set up for the assessment of the "daily food intake". To the purpose of performing an exact administration of the extracts for weight unit, three days before the start of the experiment, the animals and the food amount have been weighed so as to make a correct administration of extract for weight unit. The effect of the extract administration has been evaluated by weighing the amount, every 24 hours, of the food intake until to 72 hours after the administration of the extracts.

In the table are reported the quantities (g) of the food intake of each experimental group (mean of the food intake of the mice of each group) in the first 24 hours and in the second 24 hours after the administration of the extracts.

**TABLE 1**

| Samples | Food intake (g) in the first 24 hours after the administration of the extracts | Food intake (g) in the second 24 hours after the administration of the extracts |
|---|---|---|
| Control | 3,6±0,3 | 3,6±0,3 |
| C1 | 2,6±1,2 | 2,9±1,2 |
| C2 | 2,1±1,5* | 3,1±0,4 |
| PL | 2,2±0,6* | 2,7±0,7 |
| CELL S1 | 3,8±2,0 | 2,8±1,3 |
| CELL S2 | 2,3±1,3* | 2,5±1,3 |
| Cell L Cell T | 2,1±0,6* 2,7±1,2 | 2,4±0,8 2,9±1,2 |

| | | |
|---|---|---|
| *=p<0,05 sample vs control by Student's t-test | | |

Furthermore, the proportional decreases of the food intake of the treatment groups in comparison with the control group are reported in table 2.

**TABLE 2: The proportional decrease of the food intake of the treatment groups in comparison with the control group**

| Samples | % food intake of the treatment groups in comparison with the control group, in the first 24 hours after the administration of the extracts | % food intake of the treatment groups in comparison with the control group, in the second 24 hours after the administration of the extracts |
|---|---|---|
| Control | 0, 00% | 0, 00% |
| C1 | -27,70% | -19,40% |
| C2 | -41,60% | -13,80% |
| PL | -38,80 | -25,00% |
| CELL S1 | +5,00%% | -22,20% |
| CELL S2 | -36,11% | -30,50% |
| Cell L Cell T | -41,60% -25,00% | -33,30% -19,40% |

It can be seen that the plant commercial extract (C2 extract at a concentration of 3000 mg/kg) and the plant extract (PL extract at a concentration of 600 mg/kg) show an appetite suppressing activity of about 40% lesser in comparison with the control for up to 24 hours after the administration of the extracts. Analogous effect is shown for the S2 CELL active extract from plant cells on solid medium at a concentrotion of 3000mg/kg and the L CELL from plant cells on liquid medium at a concentration of 3000mg/kg. Also, the T CELL extract total lyophilisate of cells in suspension showed a significant appetite suppression activity for up to 24 hours after administration of the extract.

### EXAMPLE 6 - ASSAY OF APPETITE SUPPRESSING ACTIVITY OF CELL L, CELL LMW, AND CELL B ACTIVE EXTRACTS

By using active extracts from cell cultures of *Hoodia gordonii* CELL L and CELL LMW, obtained as described in examples 2, 3 and 4, an appetite suppressing activity assay, such as described in the previous example, has been performed.

The following extracts have been administered:
1) CELL L extract: extract obtained from cell culture on liquid medium administered at a dosage of 3000 mg/kg;
2) CELL LMW extract: extract, containing compounds with low molecular weight (<10KDa), obtained from cell culture on liquid medium administered at a dosage of 3000 mg/kg.
3) CELL B extract: extract, containing butanol soluble compounds, obtained from cell culture on liquid medium administered at a dosage of 800 mg/kg.

Assay has been carried out using the method described in the example 4. After the quarantine period, the animals have been divided in 4 groups of 10 animals each, put in single cages.

The assessment of the "daily food intake" has been performed as described in the example 4.

The effect of the extract administration has been evaluated by weighing the amount of the food intake until to 72 hours after the administration of the extracts.

In the table 3 are reported the quantities (g) of the food intake of each experimental group (mean of the food intake of the mice of each group) in the first 24 hours and in the second 24 hours after the administration of the extracts.

**TABLE 3**

| Samples | Food intake (g) in the first 24 hours after the administration of the extracts | Food intake (g) in the second 24 hours after the administration of the extracts |
|---|---|---|
| Control | 4,3±1,2 | 4,6±0,6 |
| CELL L | 2,8±0,6* | 4,5±0,7 |
| CELL LMW | 2,5±1,1* | 4,3±1,2 |
| CELL B | 2,1±1,2* | 4,1±1,2 |

| | | |
|---|---|---|
| *=p<0,05 sample vs control according to Student's t-test | | |

Furthermore, the proportional decreases of the food intake of the treatment groups in comparison with the control group are reported in table 4.

**TABLE 4 - The proportional decrease of the food intake of the treatment groups in comparison with the control group**

| Samples | % food intake of the treatment groups in comparison with the control group, in the first 24 hours after the administration of the extracts | % food intake of the treatment groups in comparison with the control group, in the second 24 hours after the administration of the extracts |
|---|---|---|
| Control | 0, 00% | 0,00% |
| CELL L | -34,80% | -2,17% |
| CELL LMW | -41,80% | -7,59% |
| CELL B | -51,16% | -10,80% |

As it can be seen from the obtained results, CELL L, CELL LMW and CELL B active extracts cause a great reduction of the appetite in the 24 hours after the administration. In particular, at the same concentration, it is observed that CELL LMW active extract shows greater appetite suppressing capacity with respect to CELL L and CEL LMW extracts, even if it has been administered at a far lower concentration with respect to the other extracts (800 mg/kg instead of 3000 mg/kg). This means that the active principles responsible for the appetite suppressant activity are concentrated in the butanol fraction.

### EXAMPLE 7

### COMPARISON OF APPETITE SUPPRESSING ACTIVITY BETWEEN COMMERCIAL EXTRCTS AND CELL CULTURE ACTIVE EXTRACTS

The aim of such an assay was to compare the biological activities of two commercial extracts (coming from different geographical areas) and of two cell culture active extracts (obtained from two different batches of the plant biomass). The following extracts have been administered:
1) Comm 1 extract (lot number: 500650): plant commercial extract administered at a dosage of 3000 mg/kg;
2) Comm 2 extract (lot number: 040612): plant commercial extract administered at a dosage of 3000 mg/kg;
3) CELL L1 extract: total extract from cell culture suspension (lot number: 1) administered at a dosage of 3000 mg/kg;
4) CELL L2 extract: total extract from cell culture suspension (lot number: 2) administered at a dosage of 3000 mg/kg.

Assay has been carried out using the method described in the example 4. After the quarantine period, the animals have been divided in 5 experimental groups of 10 animals each housed individually in cages.

The assessment of the "daily food intake" has been performed as described in the example 4.

The results of this assay are shown in Table 5. In the table are reported the quantities (g) of the food intake of each experimental group (mean of the food intake of the mice of each group) in the first 24 hours and in the second 24 hours after the administration of the extracts.

**TABLE 5**

| Samples | Food intake (g) in the first 24 hours after the administration of the extracts | Food intake (g) in the second 24 hours after the administration of the extracts |
|---|---|---|
| Control | 2,5±0,4 | 2,6±0,5 |
| Comm 1 extract | 1,6±0,4* | 2,2±0,4 |
| Comm 2 extract | 2,3±0,6 | 2,5±0,4 |
| CELL L1 | 1,6±0,3* | 2,1+0,6 |
| CELL L2 | 1,7±0,8* | 2,1±0,5 |

| | | |
|---|---|---|
| *=p<0,05 sample vs control by Student's t-test | | |

The proportional decreases of the food intake of the treatment groups in comparison with the control group are reported in table 6. The data are expressed as percentages.

**TABLE 6: The proportional decrease of the food intake of the treatment groups in comparison with the control group.**

| Samples | % food intake of the treatment groups in comparison with the control group, in the first 24 hours after the administration of the extracts | % food intake of the treatment groups in comparison with the control group, in the second 24 hours after the administration of the extracts |
|---|---|---|
| Comm 1 extract | -46,00% | -16,00% |
| Comm 2 extract | -8,00% | -4,00% |
| CELL 1 | -46,00% | -16,00% |
| CELL 2 | -33,00% | -20,00% |

As it is evident from the obtained results, the plant commercial extracts from plants having different geographical origin show very different biological activities (between 46% and 8% less than the control group in the first 24 hours after administration). The plant cell culture active extracts coming from two different batches show very similar activities (between 46% and 33%) and with lesser variability in comparison to commercial extracts. This tends to indicate that biological activity of the cell culture active extracts is standardized. On the contrary, the level of biological activity in plant commercial extracts is more unpredictable, as it depends on the geographical origin (not only, but also to the weather conditions, to microbial contaminations, to harvesting methods and to the storage conditions until the extraction process and other variables).

### EXAMPLE 8 - DETERMINATION OF THE ANTIFUNGAL ACTIVITY BY HOODIA GORDONII CELL L ACTIVE EXTRACT

To the purpose of detecting any antimicrobial activity of the *Hoodia gordonii* CELL L active extract, microbiological assays have been prepared, generally known as multiple dilution assays. Such assays have been performed by using the following microbial strains: *Candida albicans* ATCC 2091 and Trycophiton *rubrum* ATCC 28188.

The *Hoodia gordonii* CELL L active extract has been assayed at the concentrations of 0,3 mg/ml, 1 mg/ml and 5 mg/ml. The microbial strains for testing have been inoculated into culture-broth (in Tryptone soy broth medium) containing the substances to be assayed at the concentrations described above. In parallel with these tests, culture broths containing the microbial strain alone have been prepared (controls). The culture-broths have been incubated at 30°C for a period of 14 days. Samples of the various culture-broths have been taken at 0, 7 and 14 days, with the aim of counting the number of CFU/ml in relation to the various fungal strains, by plating the cultures on solid medium. A certain number of serial dilutions have been prepared for each sample in order to determine the microbial load of each culture-broth. Each dilution has been assayed in triplicate on solid medium. Then number of CFUs reported in the table is the mean of the counts performed on the triplicate assays. The results obtained are reported in the following table.

**TABLE 7: Effect of the CELL L active extract on the growth of Candida albicans ATCC 2091**

| Samples | T0 | T7days | T14days |
|---|---|---|---|
| | (CFU/ml) | (CFU/ml) | (CFU/ml) |
| Control | 1,2 x 10⁷ | 8,3 x 10⁸ | > 10¹⁰ |
| Extract(0,3mg/ml) | 8,0 x 10⁶ | 2,2 x 10⁷ | 2,6 x 10⁸ |
| Extract(1mg/ml) | 2,0 x 10⁶ | 1,6 x 10⁸ | 1,4 x 10⁸ |
| Extract(5mg/ml) | 2,0 x 10⁶ | 3,6 x 10⁷ | 2,8 x 10⁴ |

**TABLE 8: Effect of the CELL L active extract on the growth of Trycophiton rubrum ATCC 28188**

| Samples | T0 | T7days | T14days |
|---|---|---|---|
| | (CFU/ml) | (CFU/ml) | (CFU/ml) |
| Control | 2,2 x 10⁴ | 2,6 x 10⁵ | 3,2 x 10⁵ |
| Extract(0,3mg/ml) | 4,8 x 10³ | 2,1 x 10⁴ | 3,2 x 10⁴ |
| Extract(1mg/ml) | 2,2 x 10⁴ | 5,0 x 10⁴ | 4,8 x 10³ |
| Extract(5mg/ml) | 8,0 x 10³ | 4,2 x 10³ | 5,9 x 10² |

*Hoodia gordonii* CELL L active extract exerts at 5 mg/ml concentration a significant inhibiting action in relation to the growth of *Candida albicans* ATCC 2091 and *Tricophyton rubrum* ATCC 28188.

With regard to their biological activity (as described in the examples 5, 6 and 7), the inventive active extracts are valid tools for suppressing appetite, for promoting weight loss or maintenance of a desired body weight and for treating and/or preventing obesity.

The amount and dosage regimen of the active extracts in compositions for pharmaceutical and nutritional use is determined in the light of various factors including the purpose of administration, the age, sex and body weight.

An appropriate dosage range would be approximately 0,07 mg/Kg - 21,4 mg/Kg body weight/day, expressed in terms of dry weight of active extract. A suitable daily dosage for an average, 70 kg, human being would be of about 5 to about 1500 mg and preferably of about 100 and 1000 mg/Kg.

As reported in example 8, *Hoodia gordonii* active extracts demonstrate high fungicidal activity. This activity permits the use of such products in the topical treatment of candidiasis and in large skin diseases such as onychomycosis, tinea pedis, tinae barbae (beard ringworm) and tinea corporis (body ringworm).

Furthermore, the active extracts of the invention can be also use for the preparation of a medicament for the treatment of disorders caused by exacerbated gastric acid secretion and as coadiuvant agents in the treatment of diabetes mellitus, in particular type II.

The administration of the composition would be by oral route, topical route or parenteral route or by sublingual, across mucous membranes and rectal absorption.

Pharmaceutical, nutritional and cosmetic formulations may be made by mixing the active compounds of the invention together with well-known compounds in the pharmaceutical area. The compounds which may be included in the formulations are excipient such as cellulose, lactose, sucrose, mannitol, sorbitol, Tween 60, Polwax, cetylstearilic alcohol, TEA, glycerine, carbomer, calcium phosphate, sodium phosphate monobasic and dibasic, sodium chloride, starch, gelatin, tragacanth, metylcellulose and/or polyvinylpyrrolidone (PVP), silicic acid, silicone dioxide, talc, stearic acid, magnesium/calcium stearates, polyethylene glycol (PEG), carboxymethyl starch, cross-linked PVP, agar, alginic acid and alginate, coloring agents and flavoring agents.

Some examples of pharmaceutical and cosmetic preparations are reported in the following, for the purpose of non-limiting illustration:

### Example A - Formulations for oral use (tablets)

| | | |
|---|---|---|
| Active ingredient: | CELL B extract | 100 mg |
| Excipients: | Microcrystalline cellulose | 160 mg |
| | Starch | 28 mg |
| | Lactose | 100 mg |
| | Stearic acid | 6,0 mg |

### Example B - preparation for parenteral use

| Phial 1 | | |
|---|---|---|
| Active ingredient: | CELL B extract | 50 mg |

| Phial 2 | | |
|---|---|---|
| Excipients: | Sodium phosphate dibasic | 12 mg |
| | Sodium phosphate monobasic | 1 mg |
| | Sodium chloride | 32 mg |
| Sufficient water to make up | | to 4ml |

### Example C - Cream W/O for topical application(% or mg)

| | | |
|---|---|---|
| Active ingredient: | CELL B extract | 2 |
| Excipients: | Tween 60 | 0.5 |
| | Polwax | 2.5 |
| | Cetylstearilic alcohol | 2 |
| | Carbomer | 0.5 |
| | TEA | 0.5 |
| | Glycerine | 3 |
| | Preservatives | 1 |
| | Water to make up to | 100 |

In addition to the cell culture active extracts of the invention, the compositions may include one or more further active ingredients selected from the following: capsaicin, fatty acids especially linoleic acid and conjugated linoleic acid, glycomacropeptide, long chain triglyceride, statin, plant sterols, galactose, glucuronic acid, fibers such as pectins, guar gum, psyllium and glucan, glucomannans, glucosamine HCl, hydroxycitrate, lemon, hydroxybutirrate, medium chain tryglicerid, D-tagatose, Ephedra L., ephedrine, caffeine, theobromine, potato extract, green tea extract, epigallocatechin gallate or others catechins, bioavailable forms of iodine and selenium, plant cell culture extracts containing phenylpropanoids such as teupolioside and verbascoside, peptide D4, vitamins B, C and/or E and chromium picolinate.

Alternatively, the active extracts of the invention may be combined to plant extracts possessing appetite suppressing activity such as *Cassia nomane* extract (containing dimeric flavonoids with enzymatic inhibition activity of the lipases), *Lagerstroemia speciosa* extract (corosolic acid is the active component with a blood glucose lowering activity), *Morus alba* extract (1-deoxynojirimicine 1 is the active compound with a blood glucose lowering activity), *Coleus forskohlii* extract (forskoline is the active component inducing a thermogenesis increase by activation of adenylate cyclase enzyme) *Guaranà* extracts (containing active compounds with a thermogenesis increasing aticity).

In another aspect of the invention, there is provided a pharmaceutical and nutritional preparation for simultaneous, separate or sequential use for the treatment of obesity comprising active extracts of the invention and one or more plant extracts (as described in the previous section) for suppressing or curbing appetite.

## Claims

1. A process for the preparation of a plant cell line extract from *Hoodia gordonii,* comprising the following stages in sequence:
a)collecting of the plant biomass obtained from *Hoodia gordonii* cell culture on solid medium or liquid medium;
b)selection of the clone deriving from the cell culture with greatest biological activity;
c)homogenisation of the cells;
d)separation of the cell residues from the aqueous phase (supernatant);
e)lyophilization of the supernatant.

2. The process according to claim 1 additionally comprising, before homogenisation step c), a preceding cell separation from the culture medium.

3. The process according to claims 1 or 2, wherein said separation stage d) and said preliminary separation phase occur through gravity filtration, pressure filtration or centrifugation by porous mesh.

4. The process according to claims 2 or 3 additionally comprising, after centrifugation step d) a phase of selective tangential filtration of the supernatant.

5. The process according to claim 4, wherein the tangential filtration stage takes place in a manner to separate the compounds with molecular weight higher than 10 KDa.

6. The process according the claim 5, wherein the filtrate containing compounds with molecular weight lesser than 10 KDa, obtained through the tangential filtration stage, is lyophilised.

7. The process according the claims 2 or 3, wherein the lyophilisate, obtained after the lyophilisation step e), is extracted with methanol.

8. The process according to claim 7, wherein after the methanol extraction phase, the methanol extract is dried.

9. The process according to claim 8, wherein the dried methanol extract is extracted with n-butanol.

10. The process according to any of the claims 1 to 9, wherein the homogenisation step is performed for a period of time comprised of between 5 and 30 minutes, the centrifugation is performed at a number of RPM comprised of between 1000 and 10000, for a period of time comprised of between 2 and 30 minutes and at a temperature comprised of between 10 and 40 °C, the tangential filtration step is performed at a temperature comprised of between 10 and 40°C, using filters having a cut off comprised of between 3 and 30 KDa and at a pressure comprised of between 0,2 and 3 bar, methanol is added to lyophilisate in weight/volume ratios comprised of between 1:1 and 1:50, under reflux conditions for a period of time comprised of between 5 and 180 minutes, the butanol is added to dry extract in weight/volume ratio comprised of between 1:5 and 1:100.

11. The process according to any of the claims 1 to 10, wherein the plant cell lines, before the selection phase b), are stabilised through cultures and sub-cultures on fresh culture medium.

12. The process according to any of the claims 1 to 11, wherein the selection phase b) comprises the following steps:
i)cultivating a predetermined plant cell line stabilised for a sufficient period of time to allow the multiplication of said cell line to give substantially distinct cell clusters, on solid medium;
ii)removing said substantially distinct cell clusters from said solid medium and placing each of them in a separate liquide culture medium;
iii)cultivating each of the said substantially distinct cell clusters in said liquid culture medium for a sufficient period of time to allow the multiplication of said cell clusters, and the determination of the biological activity;
iv)performing a determination of the biological activity by each of said cell clusters in said liquid culture medium;
v)selecting the cell cluster with greatest biological activity;
vi)repeating the process cycle according to stages i), ii), iii), iv) and v) on said cell cluster, selected acoording to stage v), a sufficient number of times until the biological activity of interest produced by the selected cell cluster, and by the cell clusters deriving from further selection cycles, is essentially constant.

13. The process according to any of the claims 1 to 12, wherein following the selection stage, the plant cell line are subjected to fermentation.

14. The process according to claim 13, wherein said fermentation comprises the following stages:
A)the inoculation of said selected plant clone into liquid medium and the multiplication thereof for a sufficient period of time to obtain an increase in cellular mass of at least 300% of the weight;
B)optionally, transfer of the suspension obtained from stage A) into fresh liquid culture medium and the multiplication thereof for a sufficient period of time to obtain an increase in cellular mass of at least 300% of the weight thereof;
C)optionally, the repetition of stage B) at least one additional time;
D)the transfer of the suspension obtained in stages A), B) or C) into fresh liquid culture medium in a fermenter to give a biomass with a biological activity of interest.

15. The process according to claim 14, wherein the fermentation is conducted for a period of time of 7-40 days, preferably for 14-21 days.

16. The process according to claims 14 or 15, wherein the fermentation is conducted at a temperature comprised between of 15 and 35°C, preferably at 25°C.

17. *Hoodia gordonii* cell line extract obtainable according to any of the claims 1 to 16.

18. The extract according to claim 17, wherein the Hoodia gordonii cell line is the cell line denominated IRB HGORD42 DSM 17443.

19. The extract according to the claims 17 or 18 for use as a medicament.

20. The extract or medicament according to the claim 19 for the treatment of the obesity.

21. The extract or medicament according to claim 19 for preparing a medicament having an appetite suppressing activity.

22. The extract or medicament according to claim 19 for preparing a medicament having an anti-fungal activity.

23. The extract or medicament according to claim 19 for treatment of gastric hypersecretion.

24. The extract or medicament according to claim 19 for preparing a medicament as coadiuvant agent in the treatment of diabetes mellitus.

25. The extract or medicament according to claim 19, wherein the dosage is comprised between of 0,07 mg/Kg and 21,4 mg/Kg of body weight, expressed in terms of dry weight of active extract, and wherein the daily dosage for an average, 70 kg, human being is comprised between of 5 and 1500 mg .

26. Use of the extract obtainable according to any of the claims 1 to 16 as a cosmetic product.

27. Use of the extract obtainable according to any of the claims 1 to 16 as a nutritional product.

28. The formulations comprising the extract according to claim 18 as the active ingredient in association with pharmaceutically, nutraceutically or cosmetically acceptable excipients for human use.

29. A formulation in according to claim 28, comprising one or more plant extracts with suppressing appetite activity selected between the followings: *Cassia nomame, Lagerstroemia speciosa, Morus alba, Coleus forskohlii, Guarana.*

30. A plant cell line of *Hoodia gordonii* called IRB HGORD42 having accession number DSMZ: DSM 17443.
